Europäisches Patentamt
European Patent Office
Office européen des brevets

(19) (11) Publication number: **0 089 940**
**A1**

# (12) EUROPEAN PATENT APPLICATION

(21) Application number: 83850078.3

(22) Date of filing: 22.03.83

(51) Int. Cl.[3]: A 61 K 31/70
C 07 H 3/04, A 61 K 39/00
G 01 N 33/48

(30) Priority: 23.03.82 SE 8201852

(43) Date of publication of application:
28.09.83 Bulletin 83/39

(84) Designated Contracting States:
AT BE CH DE FR GB IT LI NL SE

(71) Applicant: Svenska Sockerfabriks AB
Box 17050
S-200 10 Malmö(SE)

(72) Inventor: Lindberg, Alf
Johan Banérs väg 46
S-182 75 Stocksund(SE)

(72) Inventor: Svensson, Sigfrid
Södra Ljungvägen 10
S-244 02 Furulund(SE)

(74) Representative: Burman, Tore et al,
Bergling & Sundbergh AB P.O. Box 7645
S-103 94 Stockholm(SE)

## (54) Compositions for therapeutic or diagnostic use containing oligosaccharides.

(57) A composition for therapeutic or diagnostic use in connection with disease-generating microorganisms, the composition containing as an active constituent a structural element having the formula:

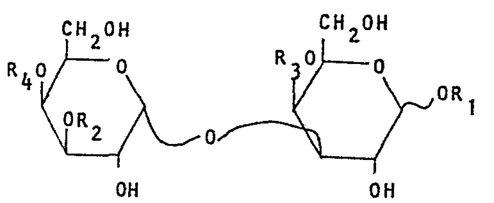

(I)

wherein $R_1$, $R_2$, $R_3$ and $R_4$ are same or different and are hydrogen or an organic residue, for example lower alkyl, lower acyl or a carbohydrate residue, or an inorganic residue, such as sulphate or phosphate, and wherein $OR_1$ is in α- or β-configuration; a process for therapeutic treatment; a process for identification or quantification of the structural element; a process for the purification of the acceptor structure of bacteria; and a process for disinfection.

EP 0 089 940 A1

Croydon Printing Company Ltd.

0089940

TITLE MODIFIED
see front page

TITLE OF INVENTION

Composition for therapeutic or diagnostic use and a process for therapeutic treatment.

The present invention relates to compositions which can be used for therapeutic treatment of infections cause by disease-generating microorganisms, such as bacteria, particularly intestinal bacteria, such as Gram-negative types. The invention is particularly applicable to bacteria of the genus Enterobacteriaceae such as Escherichia coli-bacteria, particularly K88+. The compositions according to the invention can also be used for prophylactic and diagnostic processes. The invention also relates to a process for therapeutic treatment of mammals including man. Although the invention is not limited in such a way it will in the following be illustrated essentially in connection to the bacterium E.coli.

Bacterial infection courses are often initiated by the fact that the bacterium has the ability to adhere to epithelial tissue. This adhering capacity is specific in that different bacteria adhere preferentially to different types of tissues. Thus, studies have shown that the bacterium E.coli possessing fimbriae (pili) designated K-antigen K88 adheres well to intestinal epithelium of piglets (Ref. 1). It has also been shown that the K88 fimbriae are responsible to the ability of E.coli K88+ to agglutinate guineapig erythrocytes (Ref. 2). The mutant lacking K88-fimbriae lacks ability to adhere to intestinal epithelium with the result that said E.coli bacteria cannot colonize the intestinals of pigs (Ref. 3). It has, moreover, been shown that the K88-fimbriae recognize some structure on the "brush border" membranes of the intestinal epithelium. Thus, it has been shown that certain pigs lack this structure

in the intestinal epithelium and that this fact results in said pigs being resistant to intestinal infections caused by E.coli K88+.

The present invention has for an object to provide a substance or composition having the ability of replacing the normal receptor in vivo in relation to pathogenic bacteria prone to provide infections in man and animals.

Another object of the invention is to provide such composition or substance, which, in addition to the therapeutic use, can be used also diagnostically.

A further object of the invention is to provide a process for identification and/or quantification of the receptor structure of biological materials from mammals including man.

The invention also relates to a process for purification of bacteria or acceptor structures of bacteria and process for carrying out disinfection.

The invention is particularly directed to the receptor structure of K88-fimbriated E.coli but is not limited to this type of bacterium.

In connection with studies and experiments leading to the present invention it has been found that the receptor for K88-fimbriated E.coli found on the surface of epithelial cells and erythrocytes contains a structural element having the formula:

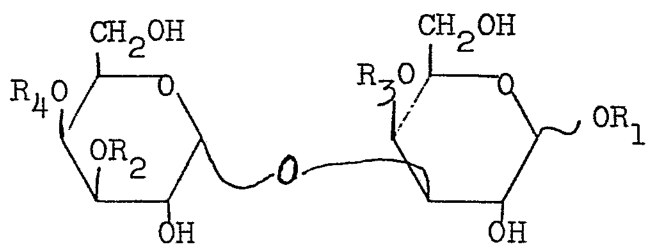

(I)

wherein $R_1$, $R_2$, $R_3$ and $R_4$ are same or different and are hydrogen or an organic residue, for example lower alkyl, lower acyl or a carbohydrate residue, or an inorganic

residue, such as sulphate or phosphate, and wherein $OR_1$ is in α- or β-configuration.

In this structural formula $R_2$, $R_3$ and $R_4$ are preferably all hydrogen. Alternatively, $R_2$ may be an α-D-Galp-residue or an α-D-Galp-(1-3)-α-D-Galp-residue.

In structural formula I $R_1$ may further be attached in either α- or β-configuration and $R_1$ can be of an arbitrary type as long as it does not adversely effect the circumstances in connection to the use of the invention.

In formula I $R_1$ may be a group having the formula:

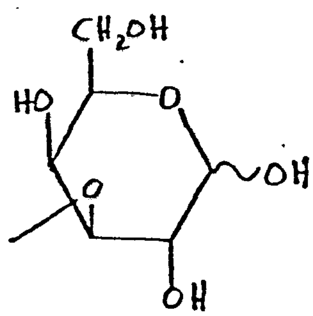

Alternatively, $R_1$ may be a group having the formula:

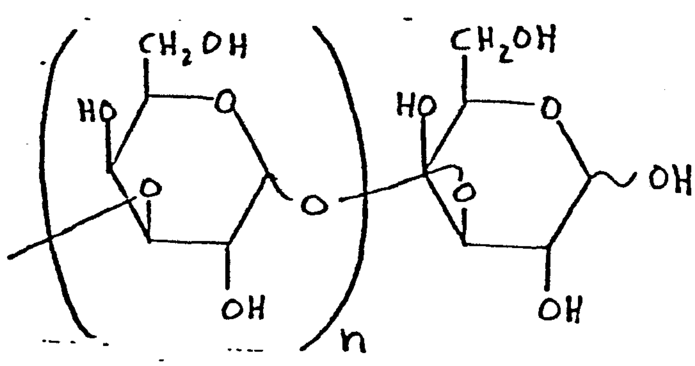

wherein n is an integer from 1-100.

The above structural element having the above formula is preferably positioned in terminal position, i.e. with the left end of the formula in freely exposed

condition. However, the structural element may be substituted with other sugar residues or organic or inorganic residues as long as they do not negatively effect the state of things in connection to the use of the composition.

The other preferred structural elements are clear from the appended patent claims.

The invention also relates to a process for therapeutic treatment of mammals including man, an active amount of a composition in accordance with the invention being administered to the mammal.

According to another aspect of the invention there is provided a composition which can be used for identification and/or quantification of the said receptor structure in biological material or to purify bacteria or their acceptor structures. The composition contains one or several structural elements according to the invention covalently or otherwise linked to for example a macromolecular carrier, optionally via a coupling arm. Useful carriers may be synthetic or naturally occurring polypeptides, polysaccharides or other types of polymers or particles.

When present the coupling arm between structural element and macromolecular carrier may be any of the following:

structural element -O-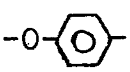 NHCSNH - macromolecular carrier

structural element -O-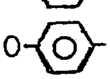 N=N-macromolecular carrier

structural element $-NH-(CH_2)_n-CH_2-$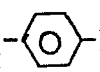-NHCSNH-macromolecular carrier

structural element $-NH(CH_2)_n-CH_2-$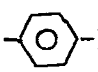-N=N-macromolecular carrier

structural element $-NH-(CH_2)_n-CONH-$macromolecular carrier

structural element -NH-macromolecular carrier

In the above examples n may vary between 1 and 15.

It should be observed that the invention is not limited to the specific coupling arms disclosed above, and the skilled artisan immediately realizes obvious modifications of those disclosed above.

The techniques for linking oligosaccharides of the type used herein to macromolecular carriers, optionally via a coupling arm, is conventional in the art, see for example References 6-10.

The haemagglutination reaction of guineapig erythrocytes caused by K88-fimbriated E.coli is due to interaction between K88-fimbriae on the surface of the bacterium and receptors on the surface of the erythrocytes containing the above-mentioned structural element.

The adherence of K88-fimbriated E.coli to "brush border" membranes is due to interaction between K88-fimbriae on the surface of the bacterium and the receptors on the surface of the membranes containing the above-mentioned structural elements.

The active constituents according to the present invention may, in a conventional manner, be formulated for use in human or veterinary medicine for therapeutic, prophylactic or diagnostic purposes. The composition or the pharmaceutical preparation may contain the active constituents in combination with the pharmaceutically acceptable carrier which may be solid, semi-solid or liquid depending on the manner of administration and other factual circumstances. The active constituents may also be used as such without addition of carrier materials. The compositions are prepared in full conformity with conventional pharmaceutical practice.

Suitable forms of the compositions of this invention include tablets, capsules, syrups, suspensions,

solutions, concentrates, reconstitutable powders in sterile forms suitable for injection or infusion. Such compositions may contain conventional pharmaceutically acceptable materials, such as diluents, binders, colours, flavours, preservatives, disintegrants and the like in accordance with conventional pharmaceutical practice in the manner well understood by those skilled in the art of formulating antibiotics.

According to a preferred aspect of the present invention the present compositions are used for therapeutic or prophylactic purposes to treat or prevent diarrhoeae in pigs, particularly piglets. For therapeutic purposes the active structure is administered suitably dissolved in the drinking water of the animal, and in particularly severe cases it can be administered through oral gavage.

For prophylactic purposes the active structure may be added directly to the animal's diet, either per se or as a concentrate in aqueous solution or other suitable formulation, for example together with a diluent to facilitate dosage.

Suitable quantities can be determined on a case to case basis and may be of the order of grams per day, for example from 0.1 to 50 g/day.

The active constituents specifically mentioned in the following disclosure are known as such and can be prepared in accordance with conventional and known processes. However, the substances have not previously been stated to possess properties making them medicinally useful.

The invention will in the following be further described by non-limiting examples.

Example 1.

Inhibition of haemagglutination of guinea pig erythrocytes with E.coli $K88^+$ or K88-fimbriae (pili) by addition of oligosaccharides.

In the tests bacteria of the type E.coli 0 149 $K88^+$ were used. The bacteria were cultivated on CFA--medium at +37°C for 18 hours. The bacterial growth was slurried in PBS, pH 7.2 to a density of 1 x $10^{10}$ bacteria/ml. K88-fimbriae were obtained in the following manner:

The bacterial strain was cultivated on Roux flasks with CFA-agar. After incubation at +37° for 24 hours the bacteria were harvested by adding 10 ml PBS/flask and sterile glass balls. The flasks were shaken and the bacterial suspension removed. The bacteria were pelletized by centrifugation (3000 x g, +4°, 30 min.). The supernatant was removed by suction. The bacteria were resuspended in 10 ml PBS and the suspension was treated in a Waring blencor 2 x 20 seconds. The suspension was centrifuged (5000 x g, +4°, 30 min.) to pelletize the bacteria. The supernatant containing fimbriae was removed by suction. The bacterial pellet is resuspended, treated in a Waring blendor and after centrifugation the supernatant is again recovered. The two supernatants are pooled and ammonium sulphate is added to a concentration of 30 %. After stirring the supernatants are allowed to stand at +4°C for 18 hours, centrifugation then taking place at 5000 x g. The supernatant was separated and the precipitation was dialyzed against PBS. The solubilized fimbriae fraction was precipitated with ammonium sulphate once more and dialyzed.

Haemagglutination is performed using erythrocytes

from guineapigs (GRBC) suspended to a concentration of 3 % v/w in PBS. The agglutination tests were carried out so that 50 µl GRBC were mixed with 2-step dilution of antigen 50 µl bacterial suspension or 50 µl fimbriae suspension and 50 µl PBS in microtitre plates. The plates were sealed with Parafilm and incubated at +44°C for 4 hours and were then recorded. The last well wherein agglutination could be read by the eye is considered as 1 haemagglutinating unit (1 HU).

In the inhibition experiments there were added instead of PBS 50 µl of an oligosaccharide solution, the inhibiting capacity was studied. The oligosaccharide was tested from the concentration 200 ug/50 µl by 2-step dilution. In the tests there were used concentrations of bacteria and fimbriae which by 3 % GRBC results in 2-4 HU. The results are presented in Table 1.

Table 1. Inhibition of haemagglutination between GRBC and K88-fimbriated bacteria or K88-fimbriae.

| Inhibitor | Minimum inhibitory concentration µg/ml |
|---|---|
| α-D-Galp-(1-3)-D-Gal | 700 |
| α-D-Galp-(1-3)-α-D-Galp-(1-3)-D-Gal | 500 |
| α-D-Galp-(1-3)-α-D-Galp-(1-3)-D-Galactitol | 700 |
| α-D-Galp-(1-3)-β-D-Galp-(1-O)-Me | 800 |
| α-D-Galp-(1-3)-α-D-Galp-(1-3)- * -α-D-Galp-(1-3)-D-Gal | 500 |
| α-D-Galp-(1-4)-D-Gal | >1500 |
| α-D-Galp-(1-4)-α-D-Galp-(1-O)-Me | >1500 |
| α-D-Galp-(1-4)-β-D-Galp-(1-O)-Me | >1500 |
| α-D-Galp-(1-4)-β-D-Galp-(1-4)-D-Glc | >1500 |
| α-D-Galp-(1-6)-D-Gal | >1500 |
| β-D-Galp-(1-6)-D-Gal | >1500 |

* Obtained by partial hydrolysis of λ-carrageenan. The oligosaccharide was purified by gel chromatography and paper chromatography.

Example 2

Preparation of oligosaccharides from GRBC inhibiting haemagglutination of GRBC with K88-fimbriated bacteria or K88-fimbriae.

For the purpose of investigating whether the active constituent tested in Example 1 had counterpart in erythrocyte tissue the following experiments were performed.

Erythrocytes from guinea pig blood are lysed and the membranes are isolated by means of centrifugation (Ref. 4). From 1 liter of blood there were obtained 12 grams of membranes.

To 10 grams of lyophilized membranes trifluoroacetic acid anhydride (200 ml) and trifluoroacetic acid (200 ml) are added, and the reaction mixture is heated at 100°C in a tube of acid-resistant, stainless steel at an overpressure of about 4 atms. for a period of time of 48 hours.

After this treatment the reaction mixture was cooled and evaporated into dryness, a dark residue being obtained. Methanol (500 ml) is added to the residue and after 30 minutes evaporation into dryness is performed. The residue is then diluted with 50 % aqueous solution of acetic acid (500 ml) and the resulting mixture is allowed to stand in room temperature for 1 hour. The reaction mixture is then filtered and evaporated into dryness.

The residue obtained is distributed between water and diethyl ether. The ether phase is washed with water 4 times and the combined aqueous phases are washed with diethyl ether 4 times. The aqueous phase obtained contains the released oligosaccharides.

The oligosaccharide mixture was reduced with an excess of sodiumborohydride in water and N-acetylated. The oligosaccharide mixture obtained was fractionated

on a Sephadex G-25 column in three different fractions. Fraction III containing mono- and disaccharides and Fraction II containing trisaccharides (mainly the carbohydrate part of asialo-GM2) did not show any inhibiting ability on the haemagglutination system GRBC och K88-fimbriae. Fraction I containing oligosaccharides larger than trisaccharides showed inhibiting ability, and chemical analysis showed that this fraction contained inter alia structural elements of the type α-D-Galp-(1-3)-D-Gal and α-D-Galp-(1-3)-α-D-Galp-(1-3)-D-Gal.

Example 3

Preparation of oligosaccharides from pig intestine inhibiting haemagglutination of GRBC with K88-fimbriated bacteria are K88-fimbriae.

Small intestine from pig was homogenized and washed with acetone, the acetone-washed material obtained being dried in air. Air-dried material (500 g) was extracted with absolute ethanol (10 l), 80 % ethanol in water (10 l) and 60 % ethanol in water (10 l). The extracts were combined and evaporated into dryness. Trifluoroacetic acid anhydride (2 l) and trifluoroacetic acid (2 l) were added to the residue, and the reaction mixture was heated at 100°C in a tube of stainless steel at an over-pressure of about 4 atms. for 48 hours.

After this period the reaction mixture was worked up according to the description of Example 2. The worked up reaction mixture containing oligosaccharides was fractionated on a Sephadex G-25-column, the mixture being divided up into three fractions I-IV. Fractions I-III contained mono- to tetra-saccharides and Fraction IV pentasaccharides and larger oligosaccharides. Only fraction IV showed inhibiting ability on haemagglutination of GRBC and K88$^+$ bacteria and K88 fimbriae, respectively. Chemical analyses of fraction IV showed that the fraction contained oligosaccharides

having the structural elements α-D-Galp-(1-3)-D-Galp and α-D-Galp-(1-3)-α-D-Galp-(1-3)-D-Gal.

Example 4

Inhibition of adherence of K88-fimbriae to brush border membranes from pig small intestine by addition of oligosaccharides.

In the experiments $I^{125}$-labelled K88 fimbriae at a concentration of 0.5 mg/ml in PBS were used. Moreover, brush border membranes prepared according to Sellwood et al at a concentration of $2 \cdot 10^6$ cells/ml in PBS were used.

Oligosaccharides tested for their inhibiting ability were dissolved in PBS to a concentration of 5 mg/ml.

In the inhibition tests 0.5 ml $I^{125}$-labelled K88 fimbriae was incubated with 0.5 ml oligosaccharide solution at 37°C for 30 minutes, 1 ml of the suspension of brush border membranes being then added. After incubation for another 60 minutes at 37°C the mixture was centrifuged for 15 minutes at 600 x g, the radioactivity being then measured in the supernatant. As a control 1 ml of PBS was used.

In these experiments it could be shown that α-D-Galp-(1-3)-D-Gal, α-D-Galp-(1-3)-α-D-Galp-(1-3)-D--Gal and α-D-Galp-(1-3)-α-D-Galp-(1-3)-α-D-Galp-(1-3)-D--Gal inhibited the adherence of K88 fimbriae to the brush border membranes to 70 %, 79 % and 73 %, respectively, whereas α-D-Galp-(1-4)-D-Gal and α-D-Galp-(1-6)--D-Gal were inactive.

Example 5

Preparation of compositions containing the structural element in at least bivalent state and covalently linked to a macromolecular carrier.

The composition was prepared starting from oligosaccharides having a free reducing end. The reactions

used are well known and they are therefore described only diagrammatically (in the scheme SR represents the structural element except for the sugar residue constituting the reducing terminal and MB represents a micromolecular carrier).

a.

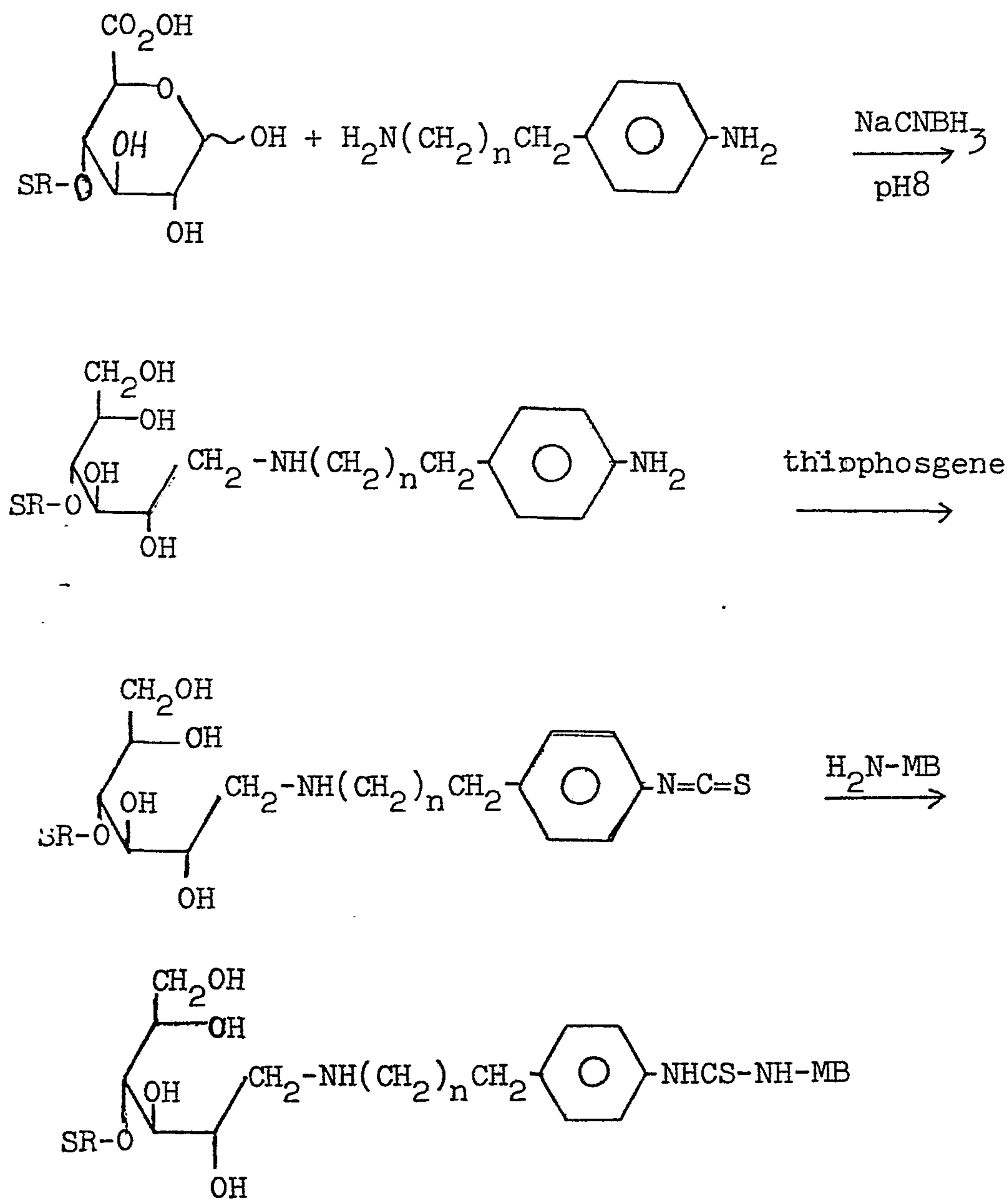

b.

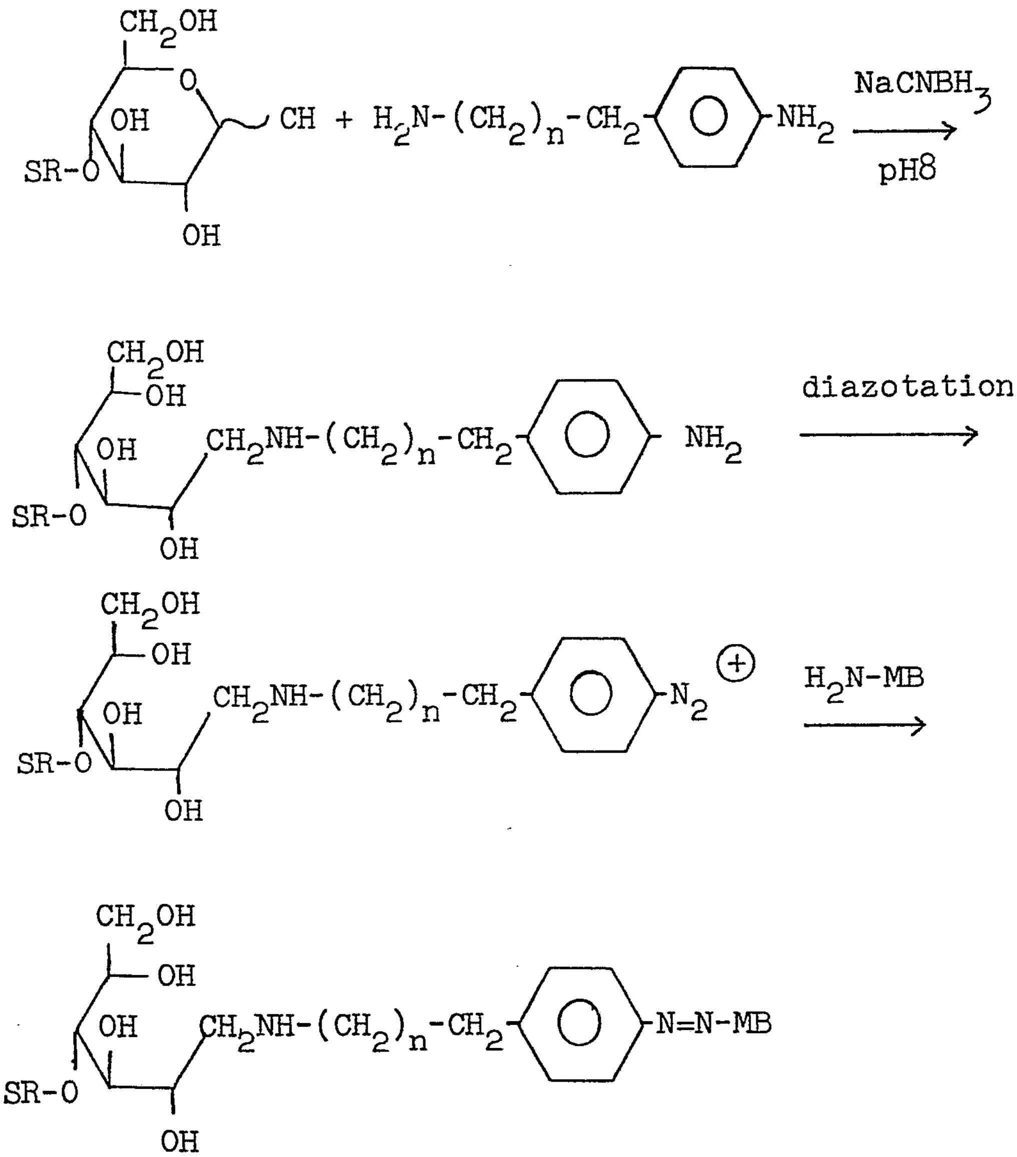


c.

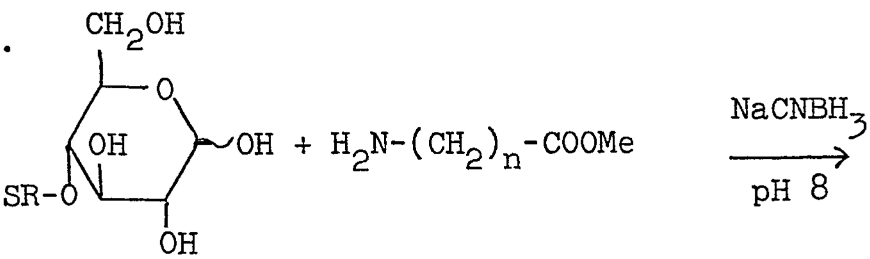

c cont.

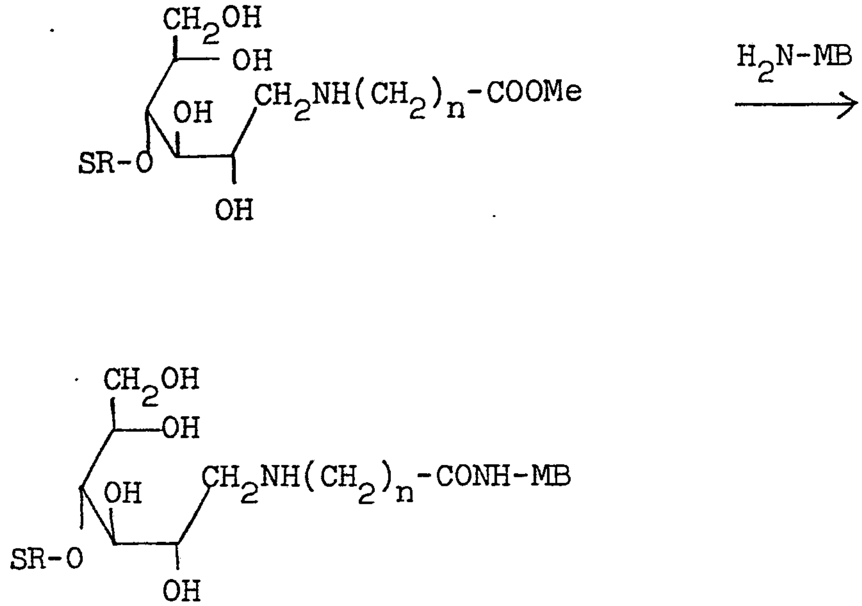


d.

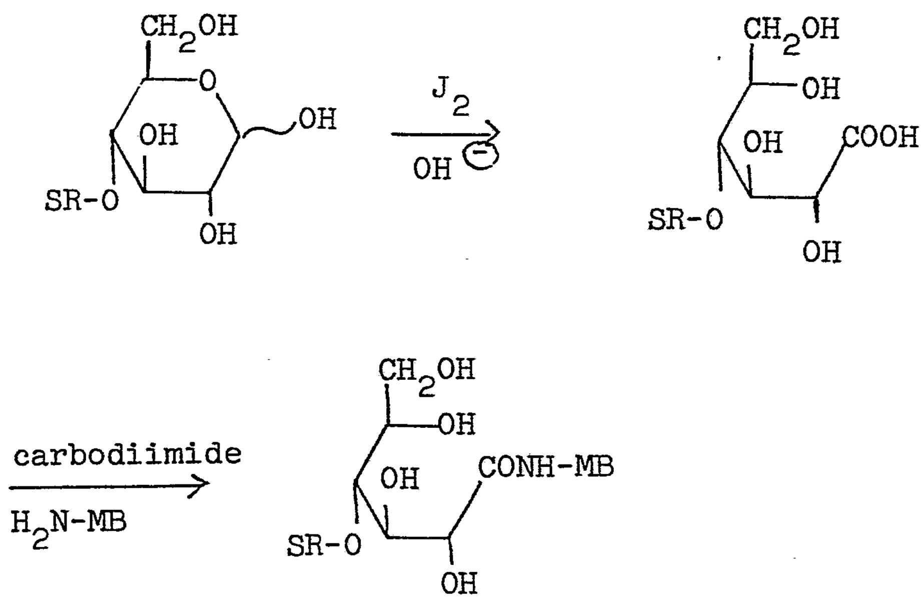


e.

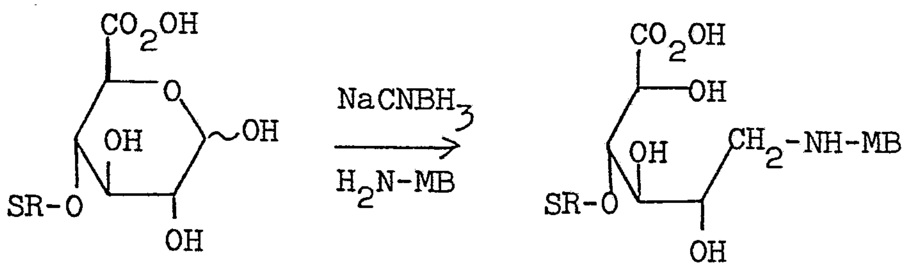

Example 6

Preparation of compositions containing the structural element according to the invention in at least bivalent association without covalent bond.

A glycolipid containing the said structural element is attached by a hydrophobic interaction to lipophilic (hydrophobic) gels, polymers or particles, for example octyl-sepharose, plastics and latex surfaces.

Example 7

Preparation of antibodies having specificity towards the said structural element.

a. Preparation of monoclonal antibodies by hybridoma technique.

I. Balb/c-mice were immunized with a composition according to the invention. The spleen from hyper-immunized animal is harvested and a cell suspension is prepared by mechanical comminution of the tissue. After gradient centrifugation to obtain a pure cell preparation the cells are fused by means of polyethylene glycol (PEG average molecular weight 1500) with established B-myeloma cell lines from Balb/c mice according to known technique. After cloning of the hybridoma cells generating the antibody desired, the cells are propagated on a large scale, the culture medium supernatants being harvested and the antibodies are being purified in a conventional manner. For identifying antibody generating clones there is used so-called enzyme-linked immunosorbent assay (ELISA) (Ref. 5).

II. Mammals are immunized with an oligosaccharide protein or polymer composition according to the invention. Antibodies are isolated from the hyperimmune serum of the mammal and purified in accordance with conventional techniques.

Example 8

Diagnostic test for identification of bacteria having acceptor structures showing specificity towards the structural element according to the invention.

a. Bacteria are incubated with guinea pig erythrocytes (GRBC) causing agglutination thereof. In a parallel experiment bacteria are incubated with guinea pig erythrocytes (GRBC) together with the structural element according to the invention at such concentration that haemagglutination is completely inhibited. Haemagglutination and inhibition of haemagglutination is performed in accordance with Example 1. Positive haemagglutination of guinea pig erythrocytes (GRBC) and complete inhibition of haemagglutination after addition of oligosaccharide verifies the fact that the bacteria possess acceptor structure.

b. Bacteria are incubated with a composition wherein the structural element in question is covalently or through other association in multivalent form linked to a particular matrix according to Example 5 or 6. Incubation is performed on microscope slides for 10-15 minutes the preparation being then studies in microscope. If the bacteria possess acceptor structure the particles are covered with bacteria. With negative reaction the particles are free from bacteria.

c. Bacteria are admixed with a composition according to Example 5 or 6 om nicroscope slides, positive reaction resulting in agglutination of particles clad with the said structural element.

Example 9

Purification of bacteria or acceptor structures

a. A composition according to Example 5 or 6 above is arranged in the form of a column. A mixture of bacteria is then passed through the column, bacteria possessing acceptor structures being held in the column

by interaction with the receptor structures of the column. After rinsing the column can be eluted with buffer containing a receptor-active structural element according to the invention and this has for an effect that the bacteria with acceptor structures are eluted in a pure form.

b. By a process fully analogous to a. the acceptor structure can be obtained in a pure form.

Bacteria or acceptor structures can be used for the manufacture of vaccins or for antibody analyses in for example body fluids, such as blood, urine or mother's milk.

Example 10

Composition for use for the purpose of disinfection

By disinfection is here primarily intended removal of bacteria from a surface, for example a wound.

A 0.1 percent by weight aqueous solution of the compound α-D-Galp-(1-3)-D-Gal is prepared and applied by means of a cotton pad onto a surface infected by *E.coli*. The solution results in effective removal of the bacterium from the surface.

When used in this disclosure the term "microorganism" is intended to cover bacteria, viruses, animal cells and plant cells.

Abbreviations:

Galp = Galactopyranosyl
Gal = Galactose
Me = $CH_3$

References:

Ref. 1. Jones, G.W. and Rutter, J.M. Infection and Immunity 6 (1972) 918-927.

Ref. 2. Jones, G.W. and Rutter, J.M. J. Gen. Microbiol. 84 (1974) 135-144.

Ref. 3. Sellwood, R., Gibbons, R.A., Jones, G.W. and Rutter, J.M. J.Med. Microbiol. 8 (1975) 405-411.

Ref. 4. Dogde, J.T., Mitchell, C. and Hanahan,D.J. Arch. Biochem. Biophys. 100 (1963) 119-130.

Ref. 5. Svenson, S.B. and K. Larsen (1977), J. Immunol, L, part C, 155.

Ref. 6. Svenson, S.B. and A.A. Lindberg (1979) J. Immunol. Meth. 25, 323.

Ref. 7. Zopf, D. et al (1978) Immunol.Meth. enzymol. L, part C, 163.

Ref. 8. Lönngren, J. et al (1976) Arch.Biochem. Biophys. 175, 661.

Ref. 9. Gray, G.R. (1978). In Meth.enzymol. L, part C, 155.

Ref. 10. Mc Broom, C.R., Samanen, C.H., Goldstein, I.J. In: Methods in Enzymology, Vol. 28B, ed. V. Ginsburg, p. 212. Academic Press, New York (1972).

0089940

PATENT CLAIMS

1. A composition for therapeutic or diagnostic use in connection with pathogenic microorganisms, characterized by containing as an active constituent a structural element having the formula:

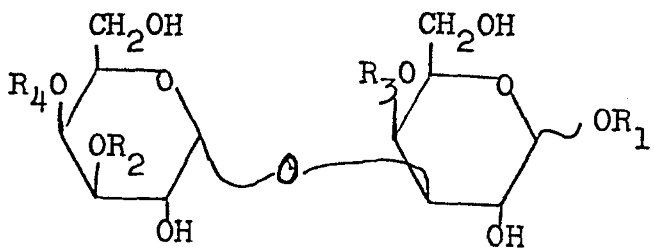

(I)

wherein $R_1$, $R_2$, $R_3$ and $R_4$ are same or different and are hydrogen or an organic residue, for example lower alkyl, lower acyl or a carbohydrate residue, or an inorganic residue, such as sulphate or phosphate, and wherein $OR_1$ is in α- or β-configuration, optionally in association with a carrier or diluent.

2. A composition according to claim 1, characterized thereby that $R_2$, $R_3$ and $R_4$ are all hydrogen.

3. A composition according to claim 1, characterized thereby that $R_2$ is an α-D-Galp residue and $R_3$ and $R_4$ are both hydrogen.

4. A composition according to claim 1, characterized thereby that $R_2$ is an α-D-Galp-(1-3)-α-D-Galp residue and $R_3$ and $R_4$ are both hydrogen.

5. A composition according to any of claims 1-4, characterized thereby that $R_1$ is hydrogen, lower alkyl, lower acyl, an inorganic residue, such as phosphate or sulphate, a carbohydrate residue or ceramide.

6. A composition according to claim 5, characterized thereby that $OR_1$ is in α-configuration.

7. A composition according to claim 6, characterized thereby that $R_1$ is a group having the formula:

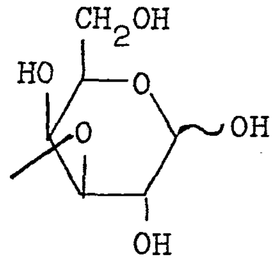

8. A composition according to claim 6, characterized thereby that $R_1$ is a group having the formula:

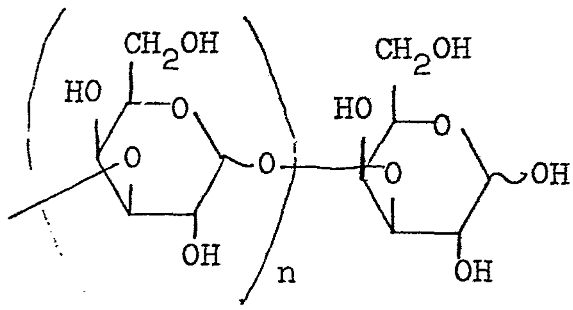

wherein n is an integer from 1-100.

9. A composition according to claim 1 for use as an inhibitor of bacterial adherence to human or animal cells.

10. A composition according to any preceding claim, characterized thereby that the active constituent originates from galactan hydrolysate.

11. A composition according to claim 10, characterized thereby that the active constituent originates from hydrolysates of $\lambda$-carrageenans.

12. A composition according to claim 10, characterized thereby that the active constituent originates from

α-D-galactans.

13. A process for therapeutic treatment of mammals including man, characterized by administering to the mammal an active amount of a composition according to any preceding claim.

14. A composition according to claims 1-12, characterized by containing the said structural element covalently linked to a macromolecular carrier.

15. A composition according to claim 14, characterized by containing said structural element covalently linked to a macromolecular carrier via a coupling atm.

16. A composition according to claim 14 or 15, characterized by using as a macromolecular carrier a synthetic or naturally occurring polypeptide, polysaccharide, other polymer or particle.

17. A composition according to claim 15 or 16, characterized thereby that the coupling arm between structural element and macromolecular carrier is:

structural element - 1 - 0 -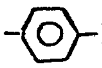-NHCSNH-macromolecular carrier,

structural element -1-0-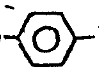-N=N - macromolecular carrier,

structural element $-NH-(CH_2)_n-CH_2-$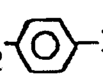-NHCSNH-macromolecular carrier,

structural element $-NH-(CH_2)_n-CH_2-$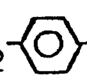-N=N-macromolecular carrier,

structural element $-NH-(CH_2)_n-CO-NH-$macromolecular carrier,

structural element -NH-macromolecular carrier,

or

structural element $-NH(CH_2)_n-CO-NH-$macromolecular carrier,

wherein n can vary between 1 and 15.

18. A composition according to claim 9 for therapeutic or prophylactic use to treat or prevent diarrhoeae in domestic animals.

19. A process for identification or quantification of the said structural element in native biological material from mammals including man, characterized by using in the process antibodies, the generation of which has been induced by the composition according to any of claims 1-12 and 14-18.

20. A process for the purification of acceptor structures of bacteria, characterized by using for the purification the affinity between the structural element of the composition according to any of claims 1-12 and 14-19 and the said acceptor structure on the bacteria.

21. A process for performing disinfection on surfaces, characterized by applying to the surface a composition according to any of claims 1-12 and 14-19 and then removing the composition with the bacteria adhering thereto.

European Patent Office

**PARTIAL EUROPEAN SEARCH REPORT**
which under Rule 45 of the European Patent Convention shall be considered, for the purposes of subsequent proceedings, as the European search report

Application number: 0089940

EP 83 85 0078

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| Y | JOURNAL OF THE CHEMICAL SOCIETY, 1979, no.7, C. AUGÉ et al.: "Stannylene Derivatives in Glycoside Synthesis. Application to the Synthesis of the Blood-group B Antigenic Determinant", pages 1825-32<br>* page 1830: Derivatives (20) and (21)<br>-- | 1,2,5 | A 61 K 31/70<br>C 07 H 3/04<br>A 61 K 39/00<br>G 01 N 33/48 |
| Y | CARBOHYDRATE RESEARCH, vol.101, 1982, Elsevier Scientific Publishing Company, AMSTERDAM (NL)<br>P.A.J. GORIN:"Preparation of α and β Anomers of Various Isomeric Methyl O-D-Galactopyranosyl-D-Galactopyranosides. Standards For Interpretation of 13C-N.M.R. Spectra of D-Galactopyranans", pages 13-20<br>./. | | **TECHNICAL FIELDS SEARCHED (Int. Cl. 3)**<br>A 61 K 31/00<br>C 07 H 3/00<br>G 01 N 33/00 |

**INCOMPLETE SEARCH**

The Search Division considers that the present European patent application does not comply with the provisions of the European Patent Convention to such an extent that it is not possible to carry out a meaningful search into the state of the art on the basis of some of the claims.

Claims searched completely: 1-12,14-21
Claims searched incompletely:
Claims not searched: 13
Reason for the limitation of the search:

Method for treatment of the human or animal body by surgery or therapy (see article 52(4) of the European Patent Convention).

| Place of search | Date of completion of the search | Examiner |
|---|---|---|
| The Hague | 04-05-1983 | VERHULST |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82

European Patent Office

**PARTIAL EUROPEAN SEARCH REPORT**

0089940
Application number

EP 83 85 0078 -2-

| Category | Citation of document with indication, where appropriate, of relevant passages | Relevant to claim | CLASSIFICATION OF THE APPLICATION (Int. Cl.) |
| --- | --- | --- | --- |
| | * page 15: Derivative (5) * | 1,6 | |
| | -- | | |
| Y | CHEMICAL ABSTRACTS, vol.56, 1962, column 6066e COLUMBUS OHIO (US) & Can. J. Chem. 39, 1563-73(1961) S. HAQ et al.: "Structure of an arabinogalactoglycan from tamarack (Larix laricina)." | | |
| | * abstract: Derivative(XIV) * | 1,6,7 | |
| | -- | | TECHNICAL FIELDS SEARCHED (Int. Cl.3) |
| Y | EP - A - 0 035 484 (KÄLLENIUS G.) | | |
| | * pages 1-4 * | 1,9-21 | |
| | -- | | |
| Y | EP - A - 0 004 823 (ANVAR) | | |
| | * pages 42-43 * | 1,9-21 | |
| | -- | | |
| Y | EP - A - 0 038 251 (ANVAR) | | |
| | * pages 26-30 * | 1,9-21 | |
| | -- | | |
| A | CHEMICAL SOCIETY REVIEWS 1978, vol. 7, no.4 R.U. LEMIEUX, F.R.S.: "Haworth Memorial Lecture. Human Blood Groups and Carbohydrate Chemistry", pages 423-52 | | |
| | * pages 423-452 * | 1 | |
| | ------- | | |

EPO Form 1505.3 05.78